# EUROPEAN PATENT SPECIFICATION

(11) **EP 3 639 731 B1**
(45) Date of publication and mention of the grant of the patent: **14.01.2026**
(21) Application number: 18745715.5
(22) Date of filing: 07.06.2018
(51) Int. Cl.: A61B 5/00, A61B 5/11

(54) **SYSTEM FOR RECOGNIZING MOVEMENT**
BEWEGUNGSERKENNUNGSSYSTEM
SYSTÈME DE RECONNAISSANCE DE MOUVEMENT

(30) Priority: 13.06.2017 PT 2017110142
(43) Date of publication of application: 22.04.2020
(73) Proprietor: Flexitex - Fabrica De Tecidos SA, 3700-257 São João Da Madeira (PT)
(72) Inventor: DA S. RODRIGUES ANDRADE, Antonio Jose, 3800-735 Aveiro (PT); OLIVEIRA FREITAS, Diana Rosa, 4520-154 Santa Maria Da Feira (PT); RODRIGUES CHAVES FERREIRA, Paulo Alexandre, 1000-138 Lisboa (PT); GUIMARAES PIMENTA, Nelson Rafael, 1000-138 Lisboa (PT)
(74) Representative: Liebl, Thomas
(86) International application number: PCT/PT2018/000010
(87) International publication number: WO 2018/231079

(56) References cited:
- US-A1- 2013 204 157
- US-A1- 2016 157 780
- US-A1- 2017 027 498
- RISICATO JEAN-VINCENT ET AL: "A Complex Shaped Reinforced Thermoplastic Composite Part Made of Commingled Yarns With Integrated Sensor", APPLIED COMPOSITE MATERIALS, KLUWER ACADEMIC PUBLISHERS, LONDON, NL, vol. 22, no. 1, 27 May 2014 (2014-05-27), pages 81 - 98, XP035446089, ISSN: 0929-189X, [retrieved on 20140527], DOI: 10.1007/S10443-014-9400-9
- GIOVANNI SAGGIO ET AL: "Resistive flex sensors: a survey", SMART MATERIALS AND STRUCTURES, IOP PUBLISHING LTD., BRISTOL, GB, vol. 25, no. 1, 2 December 2015 (2015-12-02), pages 13001, XP020292104, ISSN: 0964-1726, [retrieved on 20151202], DOI: 10.1088/0964-1726/25/1/013001

## Description

### Field of the technique

The present invention refers to the field of the systems for recognizing presence and movement of individuals on a supporting surface, in general, and in a furniture support surface, such as for example beds, in particular.

### Background of the invention

There are known several documents in prior art relating to dispositions for detecting movement of people in beds, including as monitoring means for application in hospitals and premises for accommodating senior individuals.

In particular, it is known using a disposition of the type pressure-operated electrical switch. Typically, said electrical switch is provided by the superposition of two conductors that result in contact when pressured together by the weight of an individual.

Documents US 4,263,586, US 4,295,133, disclose solutions related with this type of switch disposition.

Documents US 4,484,043 and US 6,696,653 B1 disclose a disposition of parallel conducting strips where there is a superposition between conductors that provided on a common vertical alignment along their extension.

Document EP 0671145 B1 discloses a similar disposition comprising a planar member underneath, a planar member over that deflects under the action of the weight of an individual and a sensor means placed between said planar members. This solution does not consider the measurement of variations of electrical conductivity nor provides an integration of a conducting textile disposition with a textile support.

Document US 6,987,232 B2 discloses a sensor and a method for detecting the movement of a patient, whereby the sensor uses a linearly configured resistive leader with resistors spaced apart as a means for determining at least approximately the location of a patient on the sensor, as well as an approximate length of the sensor that is compressed by the patient. The sensor comprises a top non-conducting member and a bottom non-conducting member.

Document WO 2008/104918 A1 discloses a system and method for obtaining physiologic data from a patient. Said system comprises at least one elastically deformable member, whereby said member is deformable by the patient lying in a bed. The method includes acquisition of deformation data of said member and the determination of physiological data of said patient based upon said deformation data.

Document WO 2013/003963 A1 discloses a device for detecting positional changes of people lying in beds. Said device comprises at least one elongated bridge for support of mattress and for transmission of the supported load. Said bridge comprises at least one plate with at least one section that is configured resistant to flexion. In the path of transmission of force between the load support and the support device there is disposed at least one sensor for detecting a measurement variable that can be influence by the supported load. This solution does not consider either measurement of variations of electric conductibility nor provides an integration of a conducting textile disposition with a textile support.

Saggio, G. et al.: Resistive flex sensors: a survey, Smart Materials and Structures, Vol. 25, 013001, 2016, provides a review of resistive flex sensors (RFSs). The document discusses their structural design, working principles, materials, manufacturing methods, and integration strategies. Applications include wearable motion detection, gesture recognition, medical rehabilitation, and body posture monitoring. The review further covers system-level implementations, including signal conditioning and wireless data transmission.

### Description of the invention

The objective of the present invention is to provide a system for monitoring an individual on a supporting surface, in particular on a supporting surface that comprises a textile material support, such that said system provides a simpler construction and a more reliable monitoring.

The objective above is solved according to the present invention by means of a system according to claim 1.

In particular, the monitoring system according to the present invention comprises a conducting textile disposition that presents a plurality of conducting textile yarns, and at least one control device adapted so that it can apply an electrical tension in successive moments, measure at least one parameter of electrical conductibility, comparing with at least one previously measured value in each of said conducting textile yarns, so as to identify a variation of said parameter of electric conductibility.

Said conducting textile yarns comprise a textile support and a plurality of electricity conducting particles provided so that a variation of electrical conductibility occurs when the yarn is submitted to a stretching along respective elongated extension.

In particular, it is preferred when said conducting textiles are provided as a textile material structure that presents a distribution of metallic particles provided with a given density and alignment so that they provide electrical energy conducting properties to said textile structure that vary according to the extension of said conducting textiles relative to a non-stretched situation, whereby said textile structure is of the type textile yarn.

According to the invention, said conducting textiles are adapted so that they present a measureable variation of material properties with relation to a reference unsolicited state, including an un-stretched state or a state of different stretching.

It is preferred when said conducting textiles are adapted so that, at least in an interval of stretching values, said measurable variation is a function of the deflection force to which said conducting textiles are submitted along a direction transversal to respective elongated extension.

According to the invention, said conducting textiles are adapted so that they provide a measurable variation of at least one parameter related with the electric conductibility when submitted to a deflecting force corresponding to at least 1 kg, applied upon a region at a distance of at least 10 mm of one of the ends of said conducting textiles, and when said ends of conducting textiles are substantially fixed on the textile material support with relation to the possibility of displacement along respective elongated extension.

It is further preferred when the textile structure of the textile material support is at least one of a tissue and a knitting, and said conducting textiles are of similar shape to at least some of the remaining textiles yarns used in said textile structure.

It is preferred when said conducting textiles are provided in at least one of a inwards-oriented face and a outwards-oriented face of said textile material support, at least in the proximity, preferentially adjacent to the exterior surface of said textile material support, and when said textile material supported is provided in a material and presents a thickness so that provides a support surface deformable along a direction transversal relative thereto, for example as a result of support of at least part of a body of an individual or of the action of at least one member of the body of an individual.

It is preferred when said conducting textiles are provided at distances (D) in-between of at least 10 mm, preferentially at least 20 mm, and at most 50 mm, preferentially at most 30 mm.

It is preferred when said control device is adapted so that it can control the application of at least one previously defined electrical tension and is adapted so that it can control the application of at least one previously defined electric tension between first and second ends of conducting textiles and measuring data of at least one parameter relating to the electric conductibility through each one of said conducting textiles, for example a variation of electric tension or of electric resistance on said conducting textiles.

It is preferred when said control device is adapted so that it can reference each one of said first and second conducting textiles and thereby the at least approximate location where there is measure an electric contact between said first and second conducting textiles.

It is preferred when said control device is adapted so that it applies an electric tension one by one, successively to said first conducting textiles, and in successive loops in previously defined time intervals, and when said control device is adapted so that it compares, for each of one of said conducting textiles, a parameter value of electric conductibility measured in each measurement loop with the value measured in the previous measurement loop, preferentially with the values measured in a plurality of previous measurement loops.

Alternatively, it is preferred when the control device is adapted so that it applies an electric tension to a first textile yarn extending in a first direction, and measure the electric tensions at that moment in each of the textile yarns extending in a second direction, repeating thereafter this loop successively for each one of the remaining first textile yarns extending in a first direction, whereby said control device is further adapted so that can reference the relative position of first and second textile yarns where occurs an electric voltage as result of contact between them.

It is preferred when said control device is adapted so that in the case that there is measured a variation by at least a previously defined value, preferentially occurring in at least some of said conducting textiles, then there is registered an event of entry or an exit of occupant from said textile material support.

It is preferred when said control device is adapted so that processing of measured data can consider at least one hysteresis parameter, so as to reflect the time of recovery of said conducting textiles, after a deformation as a result of submission to a force with a transversal component relative to their extension, back to a reference position.

It is preferred when said control device is adapted so that it can periodically process measurement data relating to at least one parameter of electric conductibility, collected from said conducting textile disposition so that provides at least one of:
- an indication as to the occurrence of a deflection force susceptible of corresponding to the presence of an individual on said conducting textile disposition along time;
- the evolution of the distribution of deflection force susceptible of corresponding to the evolution of position of an individual on said conducting textile disposition along time;
- the total force exerted upon on said conducting textile disposition along time.

It is preferred when said control device is provided in connection with at least one energy source, preferentially of the battery type, preferentially adapted so that it can be connected with another external energy source.

It is preferred when said control device is adapted so that it can communicate measurement data to at least one remote data means, preferentially by near field data communication means, such as for example Bluetooth or similar.

It is preferred when said control device is adapted so that it can communicate data by means of wireless data communication with first remote data means in the proximity of a cover disposition, or of a plurality thereof, and said first remote data means can communicate with second remote data means, preferentially by means WIFI, or similar.

It is preferred when said textile material support is configured as a cover element of the surface of an object that is adapted for supporting individuals, such as for example the cover of a seat, of a mattress, or similar, presenting a top part that comprises the conducting textile disposition and is provided so that can cover at least part of said object support surface, and further presenting at least one of:
- a general configuration at least partially closed so that it can collect at least partially said object inside thereof, and
- retention means so that it can retain said textile material support in a substantially fixed position with relation to said object.

### Description of the figures

The present invention shall hereinafter be explained in greater detail based upon preferred embodiments and in the attached Figures.

The Figures show, in simplified schematic representations:
- Figure 1:: diagram of a first embodiment of a monitoring unit (1) of a monitoring system according to the invention;

- Figure 2:: diagram of a plurality of monitoring units (1) included in a monitoring system according to the invention;
- Figure 3:: diagram of a preferred embodiment of conducting textile disposition (31, 32) in a textile material support (2) in a monitoring system according to the invention;
- Figure 4:: diagram of a preferred embodiment of conducting textile disposition (3) in a monitoring system according to the invention;
- Figure 5:: diagram of a preferred embodiment of a conducting textile disposition (3) of textile material support (2) in a monitoring system according to the invention;
- Figure 6:: diagram of a preferred embodiment of a conducting textile disposition (3) of textile material support (2) in a monitoring system according to the invention;
- Figure 7:: diagram of a preferred embodiment of configuration of textile material support (2) in a monitoring system according to the invention, in an open position;
- Figure 8:: diagram of a preferred embodiment of configuration of textile material support (2) in a monitoring system according to the invention, in a closed position;

- Figure 9:: diagram of a preferred embodiment of an electricity conducting disposition between a conducting textile disposition (3) and a respective control device (7) in a monitoring system according to the invention, with first connection means (4) open;
- Figure 10:: diagram of a preferred embodiment an electricity conducting disposition between a conducting textile disposition (3) and a respective control device (7) in a monitoring system according to the invention, with first connection means (4) closed.

### Description of preferred embodiments of the invention

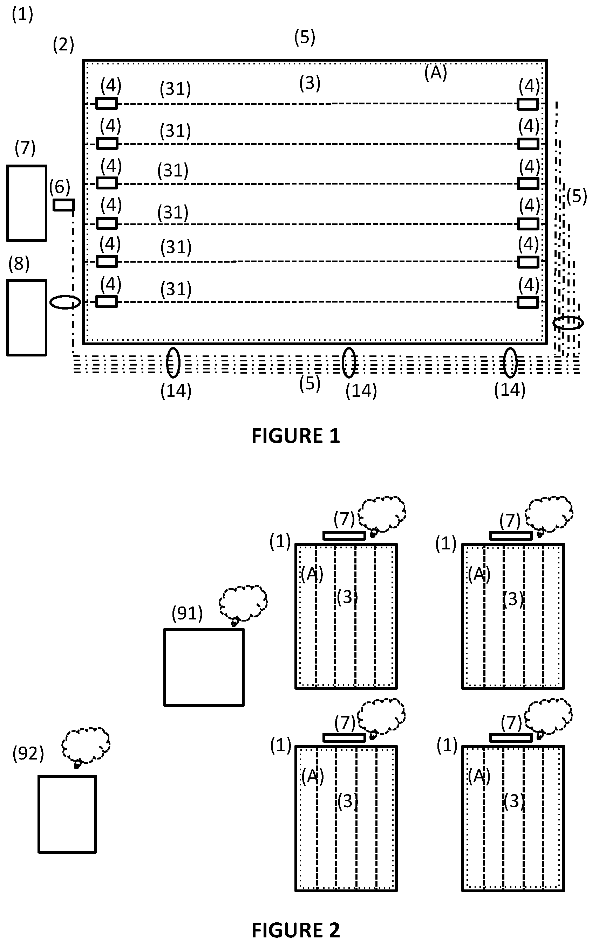

Figure 1 represents a preferred embodiment of a monitoring system according to the present invention, comprising a conducting textiles disposition (3) associated with a textile material support (2) and provided in conducting connection of electric energy with a control device (7).

The conducting textile disposition (3) comprises a plurality of conducting textiles (31) disposed along a substantially parallel direction relative to each other, and at a distance (D) between each other, whereby an electric tension can be applied between first and second ends of each one of said conducting textiles (31).

According to a first inventive aspect, said conducting textiles (31) comprise a plurality of conducting particles provided along the elongated extension of said conducting textiles. Moreover, said conducting textiles (31) are adapted so that provide a measurable variation of electric conductibility when submitted to stretching, including as a result of deflection along a direction transversal to respective elongated extension, in at least one region of respective elongated extension, so as to generate a variation of at least of distance and relative orientation between conducting particles provided on said conducting textiles (31) along the respective elongated extension.

In particular, said conducting textiles disposition (3) comprises a plurality of first conducting textiles (31) generally disposed along a first direction of said textile material support (2) and adapted so that provide a measurable variation of electric conductibility when submitted to a force presenting a component perpendicular to the support surface provided by said textile material support (2).

Said conducting textiles (31) are provided as a textile material structure that presents a distribution of metallic particles provided with a given density and alignment so that provide electric energy conducting properties to said textile structure that vary according to the stretching of said conducting textiles (31) relative to a non-stretched condition, whereby said textile structure is of the type textile yarn. The application of electric tension enable therefore measuring at least one parameter relating to the electric conductibility, for example the electric resistance, of each conducting textile (31), so that variations of said parameter in time provide an indication of load variations thereupon.

**Figure 2** represents a system comprising a plurality of monitoring units (1), for example beds, presenting respective control devices (7) provided in short range data connection, for example by means of Bluetooth, or similar, with a first remote data means (91) that can communicate data received with a second remote data means (92), for example by means of WIFI, or similar.

It is further preferred when the process of operation of a system according to the present invention further comprises the presentation of measured data, including data relating to types of events, respective time of occurrence, determined based upon periodic measurement of variations of at least one parameter of electric conductibility.

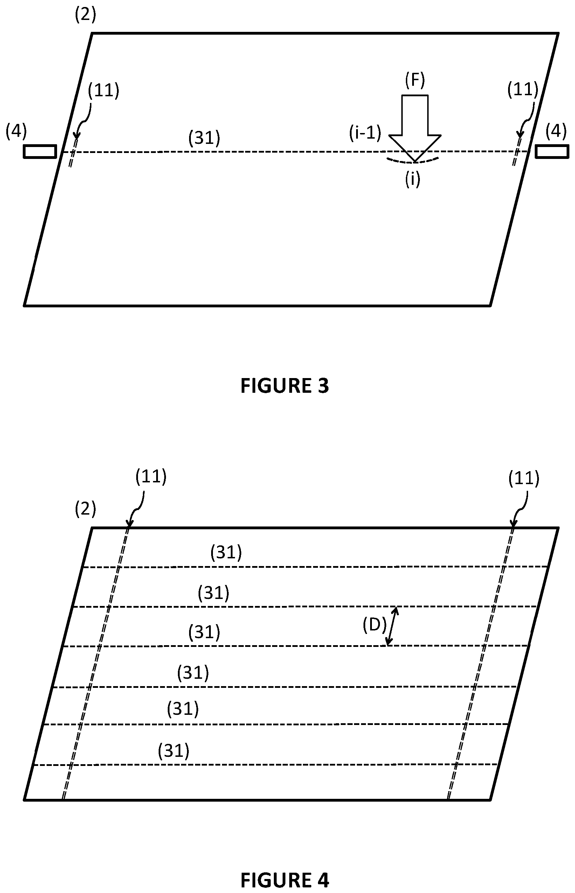

As schematically represented in **Figure 3****,** according to an inventive aspect, said control device (7) is associated to an energy source (8), and adapted so that can regulate by means of said control device (7), the realization of successive cycles i, i+1, ..., whereby each cycle comprises the application of a previously defined electric tension to each one of the conducting textiles (31) of said conducting textiles disposition (3), the measurement of at least one energy parameter related with the electric conductibility through said conducting textiles (31) and the assessment of an eventual variation in the measured value of said energy parameter with relation to at least one previous measurement.

Moreover, each monitoring cycle further includes the step of determining, based upon the value measured in a cycle i, a correspondence between at least one of:
- a variation previously measure in at least one cycle i-1 to i-n, and
- a plurality of previously measured variations in at least two cycles i-1 to i-n,
and a given type of event, including at least one of:
- entry and exit of an individual in contact with said conducting textile disposition (3);
- movement of an individual in contact with said conducting textile disposition (3).

According to a preferred embodiment, each monitoring cycle further includes determining a relative location on said conducting textile disposition (3), of a given interval of measured variations for at least one parameter associated with the electric conductibility of said conducting textile disposition (3).

Moreover, it is preferred when the control device (7) is provided so that can determine a relation between variations above a given threshold and the location of variations of at least one parameter associated to the electric conductibility on said conducting textile disposition (3), and respective evolution in time.

According to a preferred embodiment, said control device (7) is provided so that controls the application of an electric tension successively to the ends of each one of a plurality of conducting textiles (31), with a periodicity comprised between 0,001 s and 0,1 s, in ongoing manner in time.

According to a preferred embodiment (see **Figure 4**), both end regions of said conducting textiles (31) in the proximity of the limits of the top part are additionally fixed to the textile material support (2) by means of fixation means (11), provided so as to prevent the possibility of substantial displacement along the elongated extension thereof with relation to said textile material support (2). It is thereby ensure that the stretching of said conducting textiles (31) provides a reliable indication of the solicitation by a load applied upon said textile material support (2).

It is preferred when said conducting textiles are provided at distances (D) between each other of at least 10 mm, preferentially at least 20 mm, and at most 50 mm, preferentially at most 30 mm.

According to an inventive aspect, said textile material support (2) is provided so that can be applied upon said object (A) for supporting of individuals, for example a mattress, so that said conducting textiles disposition (3) is substantially retained upon said object (A), at least with relation to movements along a longitudinal extension of said object (A). Moreover, said control device (7) and electric connections between the latter and said conducting textiles disposition (3) are associated with said textile material support (2), preferentially retained thereon, and said electric connections are provided so that the placement of said textile material support (2) on said object (A) does not require establishing any electric connection, and the removal of said textile material support (2) does not require disconnecting any electric connection.

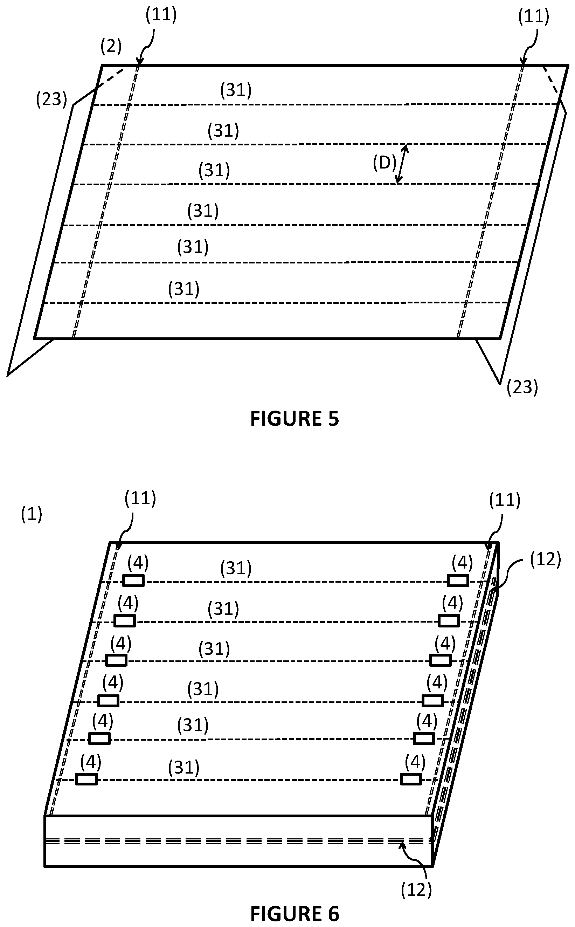

In the case of a first embodiment (1) represented in **Figure 5****,** the textile material support (2) is provided with retention means (23) provided so that can retain said textile material support (2) in a substantially fixed position with relation to said object (A).

**Figure 6** represents a second embodiment of a monitoring unit (1) where the textile material support (2) presents an at least partially closed general configuration so that can collect at least partially said object (A) inside thereof underneath said top part (21, 22) and in a substantially fixed position with relation to said textile material support (2).

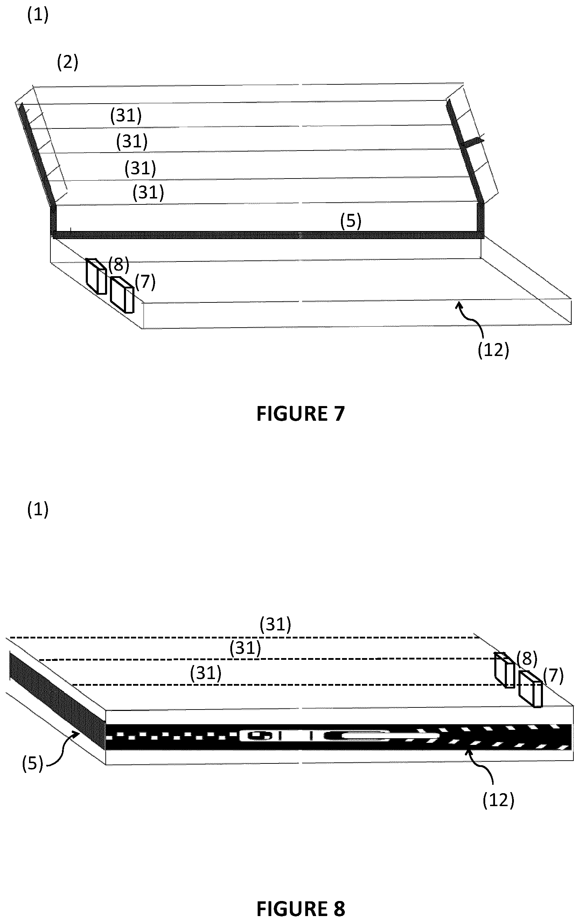

**Figures 7** and **8** represent a monitoring unit (1) corresponding to the embodiment represented in Figure 6, with a general configuration of box type, or envelope, in positions of open and closed, respectively.

In particular, it is preferred when said textile material support (2) presents support connection means (12) adapted so as to provide connection between at least two of said parts of textile material support (2), such as for example of said top part (21, 22) to the remaining parts, along at least part of a perimeter region of textile material support (2), so that said textile material support (2) can be opened, applied as envelope of a support element, and closed, and vice-versa.

It is further preferred when said textile material support (2) is provided with a general shape so as to confine said object (A) for supporting individuals inside thereof, and when said control device (7), energy source (8) and all the energy conducting connections (4, 5, 6) between said conducting textile disposition (3) and said control device (7) are provided inside of the confinement provided by said textile material support (2), so that each monitoring unit (1) does not present wire connections to the exterior, for example to external devices.

This type of configuration of monitoring unit (1) provides a rapid application upon an object (A), such as for example a mattress, without the need to carryout an installation of electric components or of establishing electric connection between thereof.

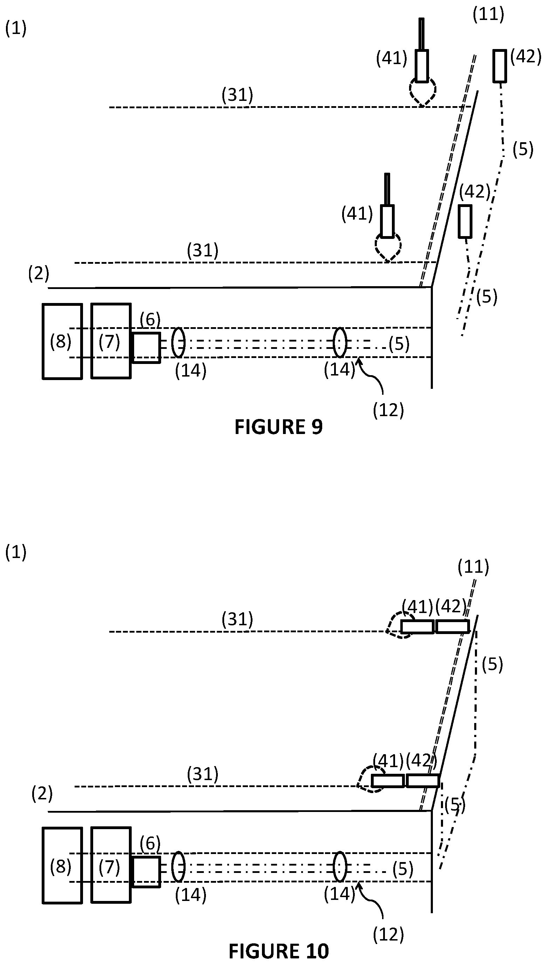

According to another inventive aspect (see **Figures 9** and **10**), the energy conducting connection between said conducting textile disposition (3) and said control device (7), comprising first connection means (4) adapted for conducting connection of a first and second end of said conducting textile disposition (3), and energy conducting disposition (5) adapted for connection of said first connection means (4) and second connection means (6) adapted for conducting connection of said energy conducting disposition (5) to said control device (7), whereby both of said first and second connection means (4, 6) are provided as pieces comprising respective conducting metallic parts. Moreover, said first connection means (4) are adapted so that can be fixed to said conducting textiles (31) by means of a clamming type of connection.

It is further preferred when said conducting textiles (31) are united to respective conducting elements of said energy conducting disposition (5), by means of first connection means (4) provided in a region of said conducting textiles (31) next to said fixation means (11), preferentially respectively on the inward side with reference to the end of the top part of textile material support (2). As further represented in Figures 9 and 10, said first connection means (4) are advantageously provided as male-female type of connections, presenting a part of conducting textile connection (31) by means of crushing, and a conducting connection part. It is preferred when the removal of said connection by crushing requires the use of a tool. It is preferred when said first connection means (4) result on the top part, next to an end thereof.

Moreover, it is preferred when said energy conducting disposition (5) passes along at least part of the extension of said textile material support and is retained along at least part of a lateral part by a plurality of retention elements (14) associated with said textile material support (2) and configured in the form of a chain-link, or similar, so as to keep the plurality of conducting yarns included on said energy conduction disposition (5) in relative proximity between each other along at least part of respective extension.

It is preferred when said energy conducting disposition (5) is retained along at least part of its extension by a plurality of retention elements (14) associated with said textile material support (2) and configured in form of a chain-link, or similar, so as to keep the plurality of conducting yarns included on said energy conducting disposition (5) in relative proximity between each other along at least part of respective extension.

An electric connection of simple construction, but reliable is herewith advantageously provided, in particular resistant to the movements by an individual on said top part of textile material support (2) that presents said conducting textile disposition (3).

## Claims

1. **System** for monitoring individuals in surfaces for supporting individuals, such as for example beds, whereby said system comprises at least one monitoring unit (1) that comprises:
- a textile material support (2) that provides a surface for supporting individuals;
- a conducting textile disposition (3) associated with said textile material support (2), said conducting textile disposition (3) comprising a plurality of conducting textiles (31), each one thereof along an elongated extension and comprising a plurality of conducting particles provided so as to provide electric conduction, said conducting textiles (31) being adapted so that they provide a measurable variation of electric conductibility in case they are submitted to stretching, including as a result of a deflection thereof along a direction transversal to respective elongated extension, in at least one region of respective elongated extension, so as to generate a variation of at least one of distance and relative orientation between said conducting particles provided in said conducting textiles (31);
- a control device (7) provided in energy conducting connection with said conducting textile disposition (3) and with an energy source (8),
**characterized**
**in that** said conducting textiles (31), provided as conducting textile yarns, are adapted to provide a measurable variation of at least one parameter related with the electric conductibility when submitted to a deflecting force corresponding to at least 1 kg, applied upon a region at a distance of at least 10 mm of one of the ends of said conducting textiles (31), and when said ends of conducting textiles (31) are fixed on the textile material support (2) with relation to the possibility of displacement along respective elongated extension.

2. System according to claim 1, **characterized in that** said conducting textiles (31) are said textile material support (2).

3. System according to claim 1 or 2, **characterized in that** said control device (7) is adapted to control the application of at least one previously defined electric tension between first and second edges of said conducting textiles (31), and the measurement of data of at least one parameter relating to the conduction of electricity through each of said conducting textiles (31), for example a variation of electrical tension or of electrical resistance in said conducting textiles (31).

4. System according to any one of claims 1 to 3, **characterized in that** said control device (7) is adapted to apply an electric tension one by one, successively to said conducting textiles (31), and in successive loops in previously defined time intervals,
and **in that** said control device (7) is adapted to compare, for each of said conducting textiles (31), a parameter value of electrical conductibility measured in each measurement loop with the value measured at least in the previous measurement loop, preferentially with the values measured in a plurality of previous measurement loops.

5. System according to any one of claims 1 to 4, **characterized in that** said control device (7) is adapted so that in case of measurement of a variation by at least a previously defined value and in a time interval of at most a previously defined value, preferentially occurring in at least some of said conducting textiles (31), then there is recorded an event of entry or of exit by an occupant of said textile material support (2) .

6. System according to any one of claims 1 to 5, **characterized in that** said control device (7) is adapted so that processing of measured data considers at least one hysteresis parameter, so as to reflect the recovery period of said conducting textiles, after deformation as a result of submission to a force with a component transversal relative to their extension, until a reference position.

7. System according to any one of claims 1 to 6, **characterized in that** said control device (7) is adapted so that it periodically processes measurement data relating to at least one parameter of electrical conductibility, collected from said conducting textile disposition (3) so that it provides at least one of:
- an indication as to the occurrence of a deflecting force susceptible of corresponding to the presence of an individual on said conducting textile disposition (3) along time;
- the evolution of the distribution of deflecting force susceptible of corresponding to the evolution of the position of an individual on said conducting textile disposition (3) in time;
- the total force exerted upon said conducting textile disposition (3) along time.

8. System according to any one of claims 1 to 7, **characterized in that** said control device (7) is provided in connection with at least one energy source (8), preferentially of the battery type, preferentially adapted so that it is connectable to another external energy source, and
**in that** it is further adapted to communicate measurement data to at least one remote data means (91, 92), preferentially by near field data communication means, such as for example Bluetooth or similar.

9. System according to claims 1 to 8, **characterized in that** said conducting textiles (31) are provided as a textile material structure that presents a distribution of metallic particles provided with a given density and alignment so that they provide electric energy conducting properties to said textile structure that vary according to the extension of said conducting textiles (31) relative to a non-stretched situation

10. System according to any one of previous claims, **characterized in that** said textile material support (2) is adapted for covering at least part of an object (A) that is adapted for supporting individuals, and further presenting at least one of:
- a general configuration at least partially closed so that it collects at least partially said object (A) inside thereof in a substantially fixed position with relation to said textile material support (2), and
- retention means (23) provided so that they retain said textile material support (2) in a substantially fixed position with relation to said object (A).

## Patentansprüche

1. System zum Überwachen von Individuen in Oberflächen zum Unterstützen von Individuen, wie zum Beispiel Betten, wobei das System zumindest eine Überwachungseinheit (1) umfasst, die umfasst:
- ein Textilmaterial-Trägerelement (2), das eine Oberfläche zum Unterstützen von Individuen bereitstellt;
- eine leitfähige Textilanordnung (3), die mit dem textilmaterial-Trägerelement (2) verbunden ist, wobei die leitfähige Textilanordnung (3) eine Vielzahl von leitfähigen Textilien (31) umfasst, jede davon entlang einer verlängerten Ausdehnung und eine Vielzahl von leitfähigen Partikeln umfassend, die so bereitgestellt sind, dass eine elektrische Leitung bereitgestellt ist, wobei die leitfähigen Textilien (31) so angepasst sind, dass sie eine messbare Variation von elektrischer Leitfähigkeit bereitstellen, falls sie einer Dehnung unterzogen werden, einschließlich als Ergebnis einer Durchbiegung derselben entlang einer Richtung quer zu der jeweiligen verlängerten Ausdehnung, in zumindest einer Region der jeweiligen verlängerten Ausdehnung, sodass eine Variation von zumindest einem aus Abstand und relativer Orientierung zwischen den leitfähigen Partikeln erzeugt wird, die in den leitfähigen Textilien (31) bereitgestellt sind;
- eine Steuervorrichtung (7), die in energieleitender Verbindung mit der leitfähigen Textilanordnung (3) und mit einer Energiequelle (8) bereitgestellt ist,
**dadurch gekennzeichnet,**
**dass** die leitfähigen Textilien (31), bereitgestellt als leitfähige textile Garne, so angepasst sind, dass sie eine messbare Variation von zumindest einem Parameter bereitstellen, der mit der elektrischen Leitfähigkeit verbunden ist, wenn sie einer Durchbiegkraft unterzogen werden, die zumindest 1 kg entspricht, angewendet auf eine Region in einer Entfernung von zumindest 10 mm von einem der Enden der leitfähigen Textilien (31), und wenn die Enden der leitfähigen Textilien (31) auf dem Textilmaterial-Trägerelement (2) fixiert sind hinsichtlich der Möglichkeit der Verschiebung entlang der jeweiligen verlängerten Ausdehnung.

2. System nach Anspruch 1, **dadurch gekennzeichnet, dass** die leitfähigen Textilien allgemein entlang einer ersten Richtung des Textilmaterial-Trägerelements (2) angeordnet sind.

3. System nach Anspruch 1 oder 2, **dadurch gekennzeichnet, dass** die Steuervorrichtung (7) angepasst ist, die Anwendung von zumindest einer zuvor definierten elektrischen Spannung zwischen ersten und zweiten Kanten der leitfähigen Textilien (31) und die Messung von Daten von zumindest einem Parameter, der die Leitung von Elektrizität durch jedes der leitfähigen Textilien (31) betrifft, zum Beispiel eine Variation der elektrischen Spannung oder des elektrischen Widerstands in den leitfähigen Textilien (31), zu steuern.

4. System nach einem der Ansprüche 1 bis 3, **dadurch gekennzeichnet, dass** die Steuervorrichtung (7) angepasst ist, eine elektrische Spannung einzeln, nacheinander auf die leitfähigen Textilien (31) und in sukzessiven Schleifen in zuvor definierten Zeitintervallen anzuwenden,
und dass die Steuervorrichtung (7) angepasst ist, für jedes der leitfähigen Textilien (31) einen Parameterwert der elektrischen Leitfähigkeit, der in jeder Messschleife gemessen wird, mit dem Wert zu vergleichen, der zumindest in der vorherigen Messschleife gemessen wurde, vorzugsweise mit den in einer Vielzahl von vorherigen Messschleifen gemessenen Werten.

5. System nach einem der Ansprüche 1 bis 4, **dadurch gekennzeichnet, dass** die Steuervorrichtung (7) so angepasst ist, dass im Falle der Messung einer Variation um zumindest einen zuvor definierten Wert und in einem Zeitintervall von höchstens einem zuvor definierten Wert, vorzugsweise auftretend in zumindest einigen der leitfähigen Textilien (31), dann ein Ereignis eines Eintritts oder eines Austritts durch einen Beleger des textilmaterial-Trägerelements (2) aufgezeichnet wird.

6. System nach einem der Ansprüche 1 bis 5, **dadurch gekennzeichnet, dass** die Steuervorrichtung (7) so angepasst ist, dass die Verarbeitung der gemessenen Daten zumindest einen Hystereseparameter berücksichtigt, sodass der Erholungszeitraum der leitfähigen Textilien nach einer Verformung als Ergebnis der Einwirkung einer Kraft mit einer Komponente quer zu ihrer Ausdehnung bis zu einer Referenzposition reflektiert wird.

7. System nach einem der Ansprüche 1 bis 6, **dadurch gekennzeichnet, dass** die Steuervorrichtung (7) so angepasst ist, dass sie periodisch Messdaten verarbeitet, die zumindest einen Parameter der elektrischen Leitfähigkeit betreffen und die von der leitfähigen Textilanordnung (3) gesammelt werden, sodass sie zumindest eines von Folgendem bereitstellt:
- eine Anzeige hinsichtlich des Auftretens einer Durchbiegkraft, die der Präsenz eines Individuums auf der leitfähigen Textilanordnung (3) entlang der Zeit entspricht;
- die Entwicklung der Verteilung der Durchbiegkraft, die der Entwicklung der Position eines Individuums auf der leitfähigen Textilanordnung (3) in der Zeit entspricht;
- die Gesamtlast, die auf die leitfähige Textilanordnung (3) entlang der Zeit ausgeübt wird.

8. System nach einem der Ansprüche 1 bis 7, **dadurch gekennzeichnet, dass** die Steuervorrichtung (7) in Verbindung mit zumindest einer Energiequelle (8) bereitgestellt ist, vorzugsweise vom Batterietyp, vorzugsweise so angepasst, dass sie mit einer anderen externen Energiequelle verbindbar ist, und dass sie weiter so angepasst ist, Messdaten zumindest einem entfernten Datenmittel (91, 92) zu übermitteln, vorzugsweise durch Nahfeld-Datenkommunikationsmittel, wie zum Beispiel Bluetooth oder Ähnliches.

9. System nach einem der Ansprüche 1 bis 8, **dadurch gekennzeichnet, dass** die leitfähigen Textilien (31) als eine textile Materialstruktur bereitgestellt sind, die eine Verteilung metallischer Partikel mit einer gegebenen Dichte und Ausrichtung präsentiert, sodass sie elektrische Energie leitende Eigenschaften für die textile Struktur bereitstellt, die sich gemäß der Ausdehnung der leitfähigen Textilien (31) relativ zu einer nicht gedehnten Situation verändern.

10. System nach einem der vorherigen Ansprüche, **dadurch gekennzeichnet, dass** das Textilmaterial-Trägerelement (2) angepasst ist, zumindest einen Teil eines Objekts (A), das angepasst ist, Individuen zu unterstützen, zu bedecken und weiter zumindest eines der folgenden präsentiert:
- eine allgemeine Konfiguration zumindest teilweise geschlossen, sodass es das Objekt (A) zumindest teilweise darin in einer im Wesentlichen festen Position relativ zum Textilmaterial-Trägerelement (2) sammelt, und
- Haltemittel (23), die so bereitgestellt sind, dass sie das Textilmaterial-Trägerelement (2) in einer im Wesentlichen festen Position relativ zum Objekt (A) zurückhalten.

## Revendications

1. Système de surveillance d'individus dans des surfaces destinées à supporter des individus, telles que par exemple des lits, ledit système comprenant au moins une unité de surveillance (1) qui comprend :
- un support en matériau textile (2) qui fournit une surface pour supporter des individus ;
- une disposition textile conductrice (3) associée audit support en matériau textile (2), ladite disposition textile conductrice (3) comprenant une pluralité de textiles conducteurs (31), chacun d'eux le long d'une extension allongée et comprenant une pluralité de particules conductrices disposées de manière à assurer une conduction électrique, lesdits textiles conducteurs (31) étant adaptés de manière à fournir une variation mesurable de la conductivité électrique dans le cas où ils sont soumis à un étirement, y compris à la suite d'un fléchissement de ceux-ci selon une direction transversale à l'extension allongée respective, dans au moins une région de l'extension allongée respective, de manière à engendrer une variation d'au moins un parmi la distance et l'orientation relative entre lesdites particules conductrices prévues dans lesdits textiles conducteurs (31) ;
- un dispositif de commande (7) disposé en connexion conductrice d'énergie avec ladite disposition textile conductrice (3) et avec une source d'énergie (8),
**caractérisé**
**en ce que** lesdits textiles conducteurs (31), fournis sous forme de fils textiles conducteurs, sont adaptés pour fournir une variation mesurable d'au moins un paramètre lié à la conductivité électrique lorsqu'ils sont soumis à une force de fléchissement correspondant à au moins 1 kg, appliquée sur une région à une distance d'au moins 10 mm de l'une des extrémités desdits textiles conducteurs (31), et lorsque lesdites extrémités des textiles conducteurs (31) sont fixées sur le support en matériau textile (2) par rapport à la possibilité de déplacement le long de l'extension allongée respective.

2. Système selon la revendication 1, **caractérisé en ce que** lesdits textiles conducteurs sont généralement disposés le long d'une première direction dudit support en matériau textile (2).

3. Système selon la revendication 1 ou 2, **caractérisé en ce que** ledit dispositif de commande (7) est adapté pour commander l'application d'au moins une tension électrique définie au préalable entre des premières et secondes bords desdits textiles conducteurs (31), et la mesure de données d'au moins un paramètre relatif à la conduction d'électricité à travers chacun desdits textiles conducteurs (31), par exemple une variation de la tension électrique ou de la résistance électrique dans lesdits textiles conducteurs (31).

4. Système selon l'une quelconque des revendications 1 à 3, **caractérisé en ce que** ledit dispositif de commande (7) est adapté pour appliquer une tension électrique un par un, successivement, auxdits textiles conducteurs (31), et en des boucles successives à des intervalles de temps définis au préalable,
et **en ce que** ledit dispositif de commande (7) est adapté pour comparer, pour chacun desdits textiles conducteurs (31), une valeur de paramètre de conductivité électrique mesurée dans chaque boucle de mesure avec la valeur mesurée au moins dans la boucle de mesure précédente, de préférence avec les valeurs mesurées dans une pluralité de boucles de mesure précédentes.

5. Système selon l'une quelconque des revendications 1 à 4, **caractérisé en ce que** ledit dispositif de commande (7) est adapté de sorte que, en cas de mesure d'une variation d'au moins une valeur définie au préalable et dans un intervalle de temps d'au plus une valeur définie au préalable, se produisant de préférence dans au moins certains desdits textiles conducteurs (31), il est alors enregistré un événement d'entrée ou de sortie par un occupant dudit support en matériau textile (2).

6. Système selon l'une quelconque des revendications 1 à 5, **caractérisé en ce que** ledit dispositif de commande (7) est adapté de sorte que le traitement des données mesurées prend en compte au moins un paramètre d'hystérésis, de manière à refléter la période de récupération desdits textiles conducteurs, après déformation résultant de la soumission à une force ayant une composante transversale par rapport à leur extension, jusqu'à une position de référence.

7. Système selon l'une quelconque des revendications 1 à 6, **caractérisé en ce que** ledit dispositif de commande (7) est adapté de sorte qu'il traite périodiquement des données de mesure relatives à au moins un paramètre de conductivité électrique, recueillies à partir de ladite disposition textile conductrice (3), de sorte qu'il fournit au moins un parmi :
- une indication quant à l'occurrence d'une force de fléchissement susceptible de correspondre à la présence d'un individu sur ladite disposition textile conductrice (3) au cours du temps ;
- l'évolution de la distribution de la force de fléchissement susceptible de correspondre à l'évolution de la position d'un individu sur ladite disposition textile conductrice (3) dans le temps ;
- la force totale exercée sur ladite disposition textile conductrice (3) au cours du temps.

8. Système selon l'une quelconque des revendications 1 à 7, **caractérisé en ce que** ledit dispositif de commande (7) est prévu en connexion avec au moins une source d'énergie (8), de préférence du type batterie, de préférence adaptée de manière à être connectable à une autre source d'énergie externe, et
**en ce qu'**il est en outre adapté pour communiquer des données de mesure à au moins un moyen de données distant (91, 92), de préférence par des moyens de communication de données en champ proche, tels que par exemple Bluetooth ou similaire.

9. Système selon les revendications 1 à 8, **caractérisé en ce que** lesdits textiles conducteurs (31) sont fournis sous forme d'une structure de matériau textile qui présente une répartition de particules métalliques prévue avec une densité et un alignement donnés, de sorte qu'elles confèrent à ladite structure textile des propriétés de conduction d'énergie électrique qui varient en fonction de l'extension desdits textiles conducteurs (31) par rapport à une situation non étirée.

10. Système selon l'une quelconque des revendications précédentes, **caractérisé en ce que** ledit support en matériau textile (2) est adapté pour recouvrir au moins une partie d'un objet (A) qui est adapté pour supporter des individus, et présente en outre au moins un parmi :
- une configuration générale au moins partiellement fermée de sorte qu'elle recueille au moins partiellement ledit objet (A) à l'intérieur de celle-ci dans une position sensiblement fixe par rapport audit support en matériau textile (2), et
- des moyens de retenue (23) prévus de sorte qu'ils retiennent ledit support en matériau textile (2) dans une position sensiblement fixe par rapport audit objet (A).
